# EUROPEAN PATENT APPLICATION

(11) **EP 2 589 343 A1**
(43) Date of publication of application: **08.05.2013**
(21) Application number: 12190785.1
(22) Date of filing: 31.10.2012
(51) Int. Cl.: A61B 10/00, A61K 49/00

(54) **Metal mixes for use in epicutaneous patch test**

(30) Priority: 02.11.2011 US 201161554669 P; 23.10.2012 US 201213658368
(71) Applicant: Smarthealth Inc., Phoenix, AZ 85008 (US)
(72) Inventor: Hamann, Curtis P., 85253 Paradise Valley, Arizona (US)
(74) Representative: BRP Renaud & Partner

(57) **Abstract**

A device and method for carrying out epicutaneous patch tests for two or more metal mixes is disclosed.

## Description

### CROSS-REFERENCE TO RELATED PATENT APPLICATIONS

This patent application claims the benefit of U.S. Provisional Patent Application No. 61/554,669, filed November 2, 2011, the entire teachings and disclosure of which are incorporated herein by reference thereto.

### FIELD OF THE INVENTION

The present invention relates generally to a form of patch able to deliver a substance to the epidermis. Specifically, the instant invention relates to a patch intended in particular for screening the sensitization state of a subject with respect to metal allergens.

### BACKGROUND OF THE INVENTION

As a group, metals are the most common Type IV allergens. Contact allergy to metals such as nickel, cobalt, and chromium is prevalent in the general population. It is estimated that up to 17% of women and 3% of men are nickel allergic, and that about 1 to 2% are allergic to cobalt, chromium, or both. Metal-induced allergic contact dermatitis (ACD) is expressed in a wide range of cutaneous reactions following prolonged or repeated exposure to personal products such as cosmetics and tattoos, detergents, jewelry and piercing, leather goods, cell phones and clothing buttons, snaps and zippers. Occupational exposure in the metal and construction industries is also a significant proportion of metal allergy reports.

ACD to metal typically occurs when the metal salts form in solution due to perspiration or exposure to body fluids. This explains why contact with stainless steel containing nickel does not usually elicit a reaction in a nickel-sensitive person, since the alloy binds the nickel so tightly that the nickel ions are not liberated during contact. If however, the exposure is prolonged, eventually allergen will penetrate the stratum corneum and ACD may result.

In addition to direct contact, ingestion or implantation of metals may result in dermatitis. Systemic contact dermatitis from nickel ingestion is well recognized most commonly causing a dyshidrosiform hand eczema. Dermatitis in any location may occur. Oral lichen planus, oral lichenoid lesions, dermatitis, joint complications and systemic reactions (e.g. Baboon Syndrome) have been reported as a result of dental/orthodontic metal allergy. Independent of the primary cause of metal sensitization, the insertion of metallic implants (e.g. intravascular stents, dental implants, cardiac pacemakers, or implanted gynecologic devices) may result in eczematous eruptions on the skin overlying the implant or in device failure caused by delayed-type hypersensitivity reactions to metals (i.e. in-stent restenosis, prosthesis loosening, inflammation, pain, oral lichen planus, granulomas, or allergic contact dermatitis). Despite repeated attempts by researchers and clinicians to further understand this difficult area of medicine, the association between metal sensitivity and cutaneous allergic reactions remains to be fully understood.

Apart from the well-known significance of nickel, cobalt and chromium in developing ACD, other metals such as aluminium, beryllium, copper, iridium, indium, mercury, palladium, platinum, rhodium, molybdenum, manganese, zinc, cadmium and titanium have been reported as emerging and rare causes of skin hypersensitivity. Patch testing is the standard of care for detection of these cutaneous reactions. For example, U.S. Patent No. 4,836,217 discloses a test strip having a plurality of patches thereon, wherein each patch contains only one metal salt as an allergen.

Today in the United States, patch test allergens are regulated as biologics by the Food and Drug Administration and require extensive development and clinical trial work prior to submission for approval. The clinical trials require "known sensitive" individuals be identified and recruited for testing to determine both the appropriate dose for the allergen as well as the safety and efficacy of the proposed product. Because the prevalence of several of these emerging metal allergens is very low in the general population, it remains a challenge to be able to locate and test known sensitive patients in a timely and efficient fashion.

The use of mixes is an efficient tool for diagnosing relatively rare Type IV sensitization (prevalence less than .05%) and has become a standard of care for other chemicals and substances such as mercaptobenzothiazoles (mercapto mix); carbamates (carba mix); black rubber mix; fragrance mix I (with 8 allergens); fragrance mix II (with 6 allergens) and the parabens (with 4-5 allergens). Although each of the individual components of these mixes are known allergens, because they elicit allergic reactions in a relatively small portion of the population, they are combined as a mix and administered to the patient as one allergen. However, to date, a metal salts mix patch test has not been employed. In general, a patch test for metals presents a variety of problems. For example, a metal tested in petrolatum requires doses of the metal which are high and often produces an irritant reaction confounding an ACD diagnosis. Also, alloys of a metal can give confounding reactions due to some synergistic effect resulting from alloying the metal. The individual metals in an alloy are also difficult to ionize, unlike a metal salt, and therefore also difficult to convert to a metal hapten since it is through a complicated mechanism that the allergic reactions occur. It is therefore desirable to provide a patch test for two or more metals to allow for a more thorough metal allergy screening.

### BRIEF SUMMARY OF THE INVENTION

In light of the foregoing, it is an object of the present invention to provide a patch test with a mix of two or more metal salts. Specifically, the mix of two or more metals provided in the patch test is in the form of two or more metal salts. It is desirable to minimize skin irritancy by using much lower metal concentrations, using a proper vehicle, and optimizing pH.

It is further an object of the invention to provide a method for determining whether an individual suffers from a metal contact allergy. The method comprises applying to the skin of an individual at least one patch having two or more metal salts as allergenic components. The metal salt mix on the patch is incorporated in a vehicle which is formulated as a film or gel adhering to the patch. The film contains at least one polymer capable of absorbing moisture which is secreted from the skin area being tested when the patch is in use. A positive test result occurs when an allergic reaction is observed on the skin.

Accordingly, it will be understood by those skilled in the art that one or more aspects of this invention can meet certain objectives, while one or more other aspects can meet certain other objectives. Each objective may not apply equally, in all its respects, to every aspect of this invention. As such, the following objects can be viewed in the alternative with respect to any one aspect of this invention.

Other objects, features, benefits and advantages of the present invention will be apparent from this summary and the following descriptions of certain embodiments, and will be readily apparent to those skilled in the art. Such objects, features, benefits and advantages will be apparent from the above as taken into conjunction with the accompanying examples, data, and all reasonable inferences to be drawn therefrom. The disclosures in this application of all articles and references, including patents, are incorporated herein by reference.

### DETAILED DESCRIPTION OF THE INVENTION

Illustrating certain non-limiting aspects and embodiments of this invention, a patch test for metal allergens is provided. The patch test comprises two or more metal salts and a vehicle.

Specifically, one embodiment of the invention is a patch test for administration of a standardized, precise dose or concentration of two or more metal salts to the skin of an individual to determine the sensitivity to each metal represented by each respective metal salt. As used herein, a "metal salt" is an ionic compound composed of a metal cation and a non-metal anion. However, oxoanions of metals can also be employed with non-metal cations, such as, for example, ammonium heptamolybdate. Unlike a pure metal or a metal alloy, a metal salt undergoes easy ionization and becomes a metal hapten (elicits an immune response when attached to a protein) upon contacting the skin or the mucous membranes of the respiratory organs.

Suitable metals used in the metal salts of the invention are those that can induce metal allergic contact dermatitis (ACD). Such metal cations include transition metals such as, for example, copper, , iron, iridium, mercury, palladium, platinum, rhodium, molybdenum, manganese, zinc, cadmium, titanium, vanadium, niobium, tin, gallium, ruthenium, tantalum, manganese, antimony, beryllium, germanium, samarium, tungsten and zirconium; alkali earth metals such as, for example, beryllium; and poor metals such as, for example, aluminium and indium.

Suitable non-metals used in the metal salts of the invention include halogens such as, for example, chloride and bromide, and other non-metals such as, for example, hydroxide, ammonium, ammoniumchloride, sulfate, oxide, acetate, nitrate, nitride, oxalate, and the like. It is to be understood by those skilled in the art that, depending on the metal salt, one or more molecules of water can be present in the metal salt, known as the "hydrate."

Accordingly, non-limiting examples of metal salts that can be used in the invention include aluminum hydroxide, aluminum chloride hexahydrate, aluminum lactate, ammonium heptamolybdate, ammonium hexachloroiridate (IV), ammonium hexachloroplatinate/dihydrogen oxide, ammonium molybdate (VI) tetrahydrate, ammonium tetrachloro platinate, ammonium tetrachloroplatinate (II)/dihydrogen oxide, cadmium chloride, calcium titanate, copper (I) oxide, copper sulfate, ferric chloride, ferrous chloride, ferrous sulfate, gallium oxide, indium (III) chloride, indium sulfate, iridium (III) chloride trihydrate, manganese chloride, mercuric chloride, elemental mercury, mercury ammoniumchloride, molybdenum (V) chloride, palladium chloride, sodium tetrachloropalladate, potassium dicyanoaurate, potassium dicyanoaurate/dihydrogen oxiderhodium chloride trihydrate, rhodium sulfate, silver nitrate, stannous chloride, stannous oxalate, tin (II) chloride, titanium (V) oxide, potassium titanium oxide oxylate, titanium nitride, titanium oxalate, vanadium (III) chloride, zinc chloride, zinc sulfate, zirconium (IV) oxide, zirconium chloride, tungstic acid, sodium salt dihydrate, ruthenium, cadmium sulfate, niobium (V)-chloride, antimony chloride, iridium(III) chloride, beryllium(II) chloride, beryllium sulfate tetrahydrate, germanium(IV) chloride, samarium(III) chloride, manganese chloride,tungsten chloride, tungsten VI sulfate, tungsten VI sulfate dihydrate, platine chloride, rhodium chloride, and the like.

In an embodiment of the invention, a film comprises a proper mix of metal salts such that the mix remains in solution rather than forming a complex and precipitating. Appropriate vehicle and pH, as well as individual concentration of each metal salt, is also considered in preparing the films of metal salt mixes.

The vehicle is selected from among substances having film-forming properties. Usually, the vehicle comprises a polymer which, together with a volatile liquid, gives a gel (hydrogel) or coalescible emulsion in which the metal salt mixes chosen can be distributed homogeneously. In a preferred embodiment of the invention, the polymer is chosen such that its gel or emulsion when spread out can dry to form a film. The vehicle should have hydrophilic properties so that it will absorb moisture when in use.

The film-forming polymers as used herein are preferably selected from among such polymers that contain multiple polar structures. Suitable examples of film-forming polymers include, but are not limited to, one or more of the following: poly (methyl methacrylates), poly (methacrylamides), polyamides, polyethyleneglycols, polypropyleneglycols, wholly alkylated polysaccharides (e.g. wholly methylated cellulose etc.), polyvinyl alcohols, and polysaccharides. Specific examples of film-forming polymers include, but are not limited to, hydroxypropyl cellulose (HPC); methylcellulose (MC); polyvinylpyrrolidone (PVP), optionally with sodium bicarbonate and sodium carbonate (PSBSC); HPC and β-cyclodextrin (HPC (β); PVP with butylhydroxyanisole (BHA) and butylhydroxytoluene (BHT) (colectively, PBB); and the like.

A "volatile" liquid, as described herein, is capable of evaporating at a temperature at which the metal salt mix employed remains in the film, without destruction of the carrier. As a rule, it is possible to employ a liquid having a relatively high vapor pressure at room temperature. Suitable examples of volatile liquids include, but are not limited to, water, ethanol and methanol, or homogeneous mixtures thereof.

The exact choice of vehicle in each case depends on the metal salt mix and the patch material employed. The metal salt mix is homogeneously distributable in the vehicle. The vehicle and patch material is chosen such that they will adhere to each other. In addition, the vehicle is a pharmaceutically acceptable, non-irritant to the skin of the subject and inert to the patch material, and does not substantially alter the allergenic and/or irritant properties of the metal salt mix. Preferably, the one or more polymers affords a film or gel that is swellable when applied occlusively, as in the case of epicutaneous testing. The resultant gel (vehicle in volatile liquid), when spread out, forms a coherent film.

Accordingly, the metal salt mix is distributed uniformly in the film-forming material. In turn, the patch material is coated reproducibly with a film of even thickness.

In another embodiment of the invention, the metal salt mix is added to a film-forming polymer dissolved or gelled in a volatile liquid. If the metal salt mix is soluble in the liquid, such as, for example, water, it may be added directly to the mixture containing the polymer. Success of the metal salt mix depends greatly on which combination of metal salts are used and the methods of manufacture and analysis. In other words, the use of one metal salt with another can effect the overall solubility of the mix in a liquid. As an alternative, the metal salt mix may be pre-dispersed in a small amount of a volatile liquid that is miscible with the liquid present in the gel. The patch material is then coated with a uniform layer of the gel which is then allowed to dry and optionally cut into a suitable number of coated patches, the latter being preferably equal in shape and size (area). It may be advantageous to have the coated patches formed with a beveled or rounded edge on their film side. The dried film may have a thickness of, for example, 0.2, 0.1, 0.05 or 0.01 mm depending on the type of coating gel. The area of the coated patches may amount to 0.2 cm². The amount of metal salt mix in the film per unit area thereof varies according to the different metal salts. Some metal salts are more potent than others, i.e. the suitable effective amount per unit area (amount necessary to elicit a reaction in sensitized persons) of each metal salt is taken into consideration. The term "effective amount per unit area" means the amount of metal salt, which when used in the test, causes an allergic reaction in most of the sensitized or normal individuals. As a rule, the amount of metal salt in each coated patch is less than 10 mg/cm², preferably less than 1 mg/cm², and more preferably less than 0.1 mg/cm². In some cases it may be desirable to determine so-called threshold values for a patient. For this purpose, coated patches are prepared which carry different amount/unit area of the same metal salt mix.

In still another embodiment, the coated patches are then placed onto a pressure-sensitive adhesive sheet material which provides a margin of at least about 5 mm, preferably at least about 1 cm projecting around each coated patch, the film-coated side of each patch being placed so as to face away from the adhesive sheet material. The coated patches are spaced apart with less than 10 cm interstices between them. The shape of each patch is not critical in the context of this invention.

The adhesive material may be permeable to moisture if the patch material is impermeable, and should be of a nature such as to gently adapt itself to the skin. To facilitate the testing procedure, it is possible to prearrange a plurality of coated patches on one common piece of the adhesive material, the individual coated patches provided with a different metal salt mix each, and/or with the same metal salt mix in different amounts per unit area. With this prearrangement of the coated patches, a test strip is obtained with which the patient is tested simultaneously against a plurality of metal salt mixes and/or respectively, against different amounts of the same metal salt mix per unit area. Such strips may comprise coated patches corresponding to a standard tray; each strip may contain up to 25, preferably up to 12 coated patches.

Accordingly, yet another embodiment of the invention is a patch test consisting of a strip of adhesive material ("self-adhesive", pressure-sensitive adhesive material) having on its adhesive face at least one test patch coated with a film with a metal salt mix incorporated therein. The patch test may be covered with a protective sheet which is peeled off immediately before use. For shipping and sale, the patch test with its protective sheet may be packaged so as to be hermetically sealed against intrusion of air, moisture, humidity and light, if desired in an inert atmosphere such as a gaseous nitrogen atmosphere.

As described herein, the resulting patch tests of the invention are used for the diagnosis of allergic contact dermatitis (ACD) in patients whose history suggests a sensitivity to one or more of the metals included on each patch. The patch tests of the invention may also be used adjunctively to evaluate other eczemas (atopic, seborrheic, venous, palmar, and plantar hyperkeratotic eczema, vesiculosis, or neurodermatitis) and other dermatologic diseases that do not heal, such as leg ulcers and psoriasis, to determine whether there may be a contact hypersensitivity component. As such, the patch tests of the invention may be indicated for patients with: persistent and recalcitrant dermatitis, suspected contact dermatitis, dorsal or patchy hand dermatitis, leg and foot dermatitis, facial dermatitis (excluding seborrheic), discoid dermatitis, dermatitis with unusual distribution, atypical allergic symptoms, and skin reactions from allergen ingestion.

### Examples

The following non-limiting examples and data illustrate various aspects and features relating to the compounds, compositions and/or methods of the present invention. In comparison with the prior art, the present compositions and methods provide results and data which are surprising, unexpected and contrary thereto. While the utility of this invention is illustrated through the preparation and use of several compositions, it will be understood by those skilled in the art that comparable results are obtainable with various other compositions as are commensurate with the scope of this invention.

All metal salts are purchased from approved suppliers. The qualities of the metal salts are those used in consumer products.

### Example 1 - Preparation of Individual Metal Salt Mix Solutions and Gels.

Each metal salt mix is dissolved or suspended in one or more solvents. Individual metal salts, concentrations of the same, correct pH, and proper solvent (volatile liquid) system/gel-forming agent are vital to the solubility of each mix. The concentration of the gel-forming agent to solvent is also very important. The compositions depicted in Tables 1-5 below have been found to be suitable metal salt mixes. The solvent is either water, an organic solvent such as ethanol, or mixtures of the two. To the metal mix solution is added either a gel-forming agent or a finished gel. The gel-forming agent is preferably HPC or PVP. The gel, together with the solvent, is also called the vehicle for the metal salt mix.

**Table 1 (Metal Mix 1 - 7.5% HPC in Water)**

| **Metal Salt** | **Concentration (mg/cm²)** |
|---|---|
| Cadmium chloride | 0.080 |
| Zinc chloride | 0.080 |
| Manganese chloride | 0.080 |
| Ammonium heptamolybdate | 0.080 |
| Ammonium tetrachloro platinate | 0.080 |
| Tin chloride | 0.080 |

**Table 2 (Metal Mix 2 - 7.5% HPC in Water)**

| **Metal Salt** | **Concentration (mg/cm²)** |
|---|---|
| Cadmium chloride | 0.080 |
| Zinc chloride | 0.16 |

**Table 3 (Metal Mix 3 - 7.5% HPC in Water)**

| **Metal Salt** | **Concentration (mg/cm²)** |
|---|---|
| Copper sulfate | 0.080 |
| Indium sulfate | 0.080 |
| Rhodium sulfate | 0.020 |

**Table 4 (Metal Mix 3 - 10.0% HPC in Water)**

| **Metal Salt** | **Concentration (mg/cm²)** |
|---|---|
| Copper sulfate | 0.080 |
| Indium sulfate | 0.040 |
| Rhodium sulfate | 0.020 |

**Table 5 (Metal Mix 3 - 7.5% HPC in Water)**

| **Metal Salt** | **Concentration (mg/cm²)** |
|---|---|
| Copper sulfate | 0.039 |
| Indium sulfate | 0.021 |
| Rhodium sulfate | 0.005 |

It is noted that for Metal Mix 2, a 5% HPC in water metal salt mix produced an unacceptable final product. Also, with respect to Metal Mix 3, while the concentrations provided in Tables 3-5 all produced acceptable results, the concentrations of Table 5 are preferable. As is evident herein, significant experimentation is necessary to obtain the correct metal salt mixes.

### Example 2 - Coating of Gels for Strip Preparation.

The individual strips are made by coating of the gels containing the metal salt mix on polyester sheets followed by removal of the solvent by drying. The coating process is preferably done on a coating machine. The gel containing the metal salt mix is spread out in front of a wired bar which, at a constant speed and pressure, is carried along the polyester sheet. To guarantee a uniform and reproducible coating, the wired bar is set a certain distance away from the surface to achieve applying the required amount of metal salt mix on the polyester surface. The solvent is evaporated from the gel coated polyester sheet in an oven.

Optionally, before the coating of the gel on the polyester sheet, the surface of the polyester is altered in such a way that a good attachment between the film containing allergen and the polyester is obtained. Preferably, corona treatment of the surface is performed.

The disclosures of all articles and references, including patents, are incorporated herein by reference. The invention and the manner and process of making and using it are now described in such full, clear, concise and exact terms as to enable any person skilled in the art to which it pertains, to make and use the same. All references cited in this specification are incorporated herein by reference. It is to be understood that the foregoing describes preferred embodiments of the present invention and that modifications may be made therein without departing from the spirit or scope of the present invention.

## Claims

1. A patch test for metal allergens comprising two or more metal salts homogenously dispersed in a vehicle, wherein the vehicle comprises a polar polymer.

2. A patch test acording to claim 1, wherein the polar polymer is selected from the group consisting of poly (methyl methacrylates), poly (methacrylamides), polyamides, polyethyleneglycols, polypropyleneglycols, wholly alkylated polysaccharides, polyvinyl alcohols, polysaccharides and combinations thereof.

3. A patch test according to claim 4, wherein the polar polymer is selected from the group consisting of hydroxypropyl cellulose, methylcellulose, polyvinylpyrrolidone, hydroxypropyl cellulose with β-cyclodextrin, polyvinylpyrrolidone with butylhydroxyanisole and butylhydroxytoluene.

4. A patch test according to claim 1, wherein each metal of the two or more metal salts is independently selected from the group consisting of copper, iron, iridium, mercury, palladium, platinum, rhodium, molybdenum, manganese, zinc, cadmium, titanium, vanadium, niobium, tin, gallium, ruthenium, tantalum, manganese, antimony, beryllium, germanium, samarium, tungsten, zirconium, an alkali earth metal, aluminium and indium.

5. A patch test according to claim 4, wherein each non-metal of the two or more metal salts is independently selected from the group consisting of halogens, hydroxide, ammonium, ammoniumchloride, sulfate, oxide, lactate, acetate, nitrate, nitride and oxalate.

6. A patch test according to claim 5, wherein the two or more metal salts are selected from the group consisting of aluminum hydroxide, aluminum chloride hexahydrate, aluminum lactate, ammonium heptamolybdate, ammonium hexachloroiridate (IV), ammonium hexachloroplatinate/dihydrogen oxide, ammonium molybdate (VI) tetrahydrate, molybdenum(V) chloride, ammonium tetrachloro platinate, ammonium tetrachloroplatinate (II)/dihydrogen oxide, cadmium chloride, calcium titanate, copper (I) oxide, copper sulfate hexahydrate, ferric chloride, ferrous chloride, ferrous sulfate, gallium oxide, indium (III) chloride, indium sulfate, iridium (III) chloride trihydrate, manganese chloride, manganese oxide, mercury ammonium chloride, molybdenum (V) chloride, palladium chloride, sodium tetrachloropalladate,potassium titanium oxide oxylate, rhodium chloride trihydrate, rhodium sulfate, silver nitrate, stannous chloride, stannous oxalate, tin (II) chloride, titanium (V) oxide, titanium nitride, titanium oxalate, vanadium (III) chloride, vanadium pentoxide, zinc chloride, zinc sulfate, zirconium (IV) oxide, zirconium chloride, tungstic acid, sodium salt dihydrate, ruthenium, cadmium sulfate, niobium (V)-chloride, antimony chloride, iridium(III) chloride, beryllium(II) chloride, beryllium sulfate tetrahydrate, germanium(IV) chloride, samarium(III) chloride, manganese chloride,tungsten chloride, tungsten VI sulfate, tungsten VI sulfate dihydrate, platine chloride and rhodium chloride.

7. A patch test according to claim 1, wherein the two or more metal mixes are homogenously dispersed in the vehicle in a state selected from the group consisting of dissolved, crystallized, micronized, emulsified or dispersed.

8. A patch test according to claim 7, wherein the two or more metal mixes are homogenously dispersed in the vehicle by dissolving the two or more metal mixes in a solution of the polar polymer and a volatile liquid and then evaporating the volatile liquid.

9. A patch test according to claim 8, wherein the volatile liquid is selected from the group consisting of water, an organic solvent and a homogenous mixture thereof.

10. A patch test according to claim 9, wherein the organic solvent is selected from the group consisting of methanol and ethanol.

11. A patch test according to claim 8, wherein the polar polymer is hydroxypropyl cellulose and the volatile liquid is water.

12. A patch test according to claim 11, wherein the two or metal salts are a mixture of cadmium chloride, zinc chloride, manganese chloride, ammonium heptamolybdate, tin chloride and ammonium tetrachloro palatinate.

13. A patch test according to claim 11, wherein the two or metal salts are a mixture of copper sulfate, indium sulfate and rhodium sulfate.

14. A patch test according to claim 8, wherein the patch is coated after the volatile liquid is evaporated.

15. A method for determining whether an individual suffers from a metal contact allergy comprising applying to the skin of an individual at least one patch according to claim 1 and observing any allergic reaction.
